# FASCICULE DE BREVET EUROPEEN

(11) **EP 0 739 201 B1**
(45) Date de publication et mention de la délivrance du brevet: **01.12.1999**
(21) Numéro de dépôt: 95907683.7
(22) Date de dépôt: 12.01.1995
(51) Int. Cl.: A61K 31/415, A61K 9/50

(54) **MICROGRANULES DE DERIVES DE 5-NITRO IMIDAZOLE**
MIKROGRANULATE ENTHALTEND 5-NITROIMIDAZOLDERIVATE
MICROGRANULES OF 5-NITRO IMIDAZOLE DERIVATIVES

(30) Priorité: 14.01.1994 FR 9400394
(43) Date de publication de la demande: 30.10.1996
(73) Titulaire: LABORATOIRES DES PRODUITS ETHIQUES ETHYPHARM, 78550 Houdan (FR)
(72) Inventeur: LEDUC, Gérard, F-45330 Malesherbes (FR); DEBREGEAS, Patrice, F-75007 Paris (FR)
(74) Mandataire: Ahner, Francis
(86) Numéro de dépôt international: FR9500039
(87) Numéro de publication internationale: WO9519168

(56) Documents cités:
- EP-A- 0 206 625
- EP-A- 0 348 808
- WO-A-91/17744
- CHEMICAL ABSTRACTS, vol. 94, no. 12, 23 Mars 1981, Columbus, Ohio, US; abstract no. 90226, & INDIAN J.PHARM.SCI., vol.42, no.2, 1980 pages 48 - 51 M.R.BAICHWAL ET. AL. 'MICROENCAPSULATION OF METRONIDAZOLE'

## Description

La présente invention concerne une nouvelle forme galénique de dérivés de 5-nitro imidazole efficace pour le traitement des parasitoses et des infections de l'ensemble du tractus gastro-intestinal.

Elle concerne plus particulièrement une nouvelle forme galénique destinée au traitement des parasitoses et des infections présentant des formes résistantes dans la partie basse du tractus intestinal, notamment au niveau du colon du sigmoïde et du rectum.

Des parasitoses des intestins telles que l'amibiase, parasitose colique qui peut se compliquer de localisations extra-intestinales, présentent dans la partie basse des intestins des formes résistantes responsables de la transmission de ces affections.

Dans le cas de l'amibiase,il s'agit des kystes d'*Entamoebacter histolytica*. Ces kystes présents dans la partie basse du tube digestif des sujets infectés, sont excrétés avec les matières fécales. Le manque d'hygiène personnelle et collective assure une propagation de la maladie ainsi qu'une auto-réinfectation des individus porteurs de ces parasites.

L'*Entamoebacter histolytica* existe également sous une forme plus labile, *Entamoebacter histolytica minuta*, laquelle n'entraîne aucune lésion mais peut se transformer en une forme pathogène, *Entamoebacter histolytica histolytica*, qui pénètre la muqueuse intestinale et entraîne la propagation de la maladie, tant dans la partie haute des intestins que la partie basse, sous forme de kystes. Cette forme est également à l'origine des complications plus générales de l'amibiase, migrant dans le foie, voire les poumons ou le cerveau.

Il est possible de traiter d'une manière rapide les parasitoses et infections du tractus intestinal par des médicaments tels qu' employés pour le traitement de la *tourista*. Toutefois, ces traitements ne pouvant être appliqués d'une manière systématique, ils ne prennent pas en compte les problèmes d'hygiène responsables de la propagation de ces maladies.

De même, il existe des traitement courants et économiques de l'amibiase par des dérivés azolés, en particuliers des dérivés de 5-nitro imidazole (métronidazole, secnidazole, etc). Toutefois, ces traitements efficaces à l'encontre des affections de la partie haute des intestins à l'encontre des formes *minuta* et *histolytica* sont peu efficaces pour le traitement des kystes. Par ailleurs, ces dérivés de 5-nitro imidazole administrées en quantités trop importantes sont absorbés dans la partie haute du tube digestif, pouvant entraîner des effets secondaires désagréables (goût métallique dans la bouche), voire toxiques.

D'autres affections développant des formes résistantes dans la partie basse des intestins, peuvent être également traitées avec les mêmes inconvénients par les dérivés de 5-nitro imidazole. Il s'agit par exemple des infections par *Helicobacter pilori*, bactérie présente dans l'estomac et dans la partie aval du tube digestif et mise en cause dans la pathogenèse de la gastrite, de l'ulcère gastrique et duodénal, l' irritation provoquée par *Helicobacter* entraînant une hypersécrétion acide de l'estomac aboutissant à une ulcération des membranes gastriques et duodénales. Sa participation a été également évoquée dans la genèse des cancers digestifs.

Compte tenu de l'importance croissante des parasitoses et des infections de l'ensemble du tractus gastro-intestinal, il devenait important de trouver un traitement économique prenant en compte les problèmes de transmission des affections liées au manque d'hygiène, c'est à dire permettant de traiter efficacement les formes résistantes présentes dans la partie basse des intestins.

De manière à résoudre ces différents problèmes, la présente invention concerne donc une nouvelle forme galénique de dérivés de 5-nitro imidazole.
La forme selon l'invention comprend une association de microgranules de dérivés de 5-nitro imidazole, consistant d'une part en des microgranules gastro-résistants et d'autre part en des microgranules à libération prolongée.

Les microgranules gastro-résistants sont destinés à assurer l'efficacité de la forme selon l'invention essentiellement dans la partie haute du tractus gastro-intestinal, alors que les microgranules à libération prolongée agissent essentiellement dans sa partie basse.

Le rapport des différents microgranules sera approprié à l'effet recherché, favorisant une action de la forme selon l'invention essentiellement dans la partie basse ou haute du tractus, ou encore de manière à assurer une action constante sur la totalité de sa longueur.Il dépendra également des excipients employés pour la préparation des microgranules.

Le rapport pondéral microgranules gastro-résistants/microgranules à libération prolongée est compris entre 6/4 et 1/9, avantageusement compris entre 4/6 et 1/9. De manière préférentielle, pour assurer une bonne efficacité de la forme selon l'invention sur les affections de la partie basse des intestins, le rapport microgranules gastro-résistants/microgranules à libération prolongée est compris entre 25/75 et 15/85.

De manière à assurer une bonne efficacité de la forme selon l'invention, les microgranules associés doivent répondre aux profils de dissolution suivants :
- microgranules gastro-résistants :
   - 2 heures dans HCl 0,1 N: < à 15%
   - 1 heure à pH 6,0: > à 75 %
- microgranules à libération prolongée :
   - 2 heures dans HCl 0,1 N: < à 15 %
   - 1 heure à 6,8 ≤ pH ≤ 7,5: < à 50%
   - 4 heures à 6,8 ≤ pH ≤ 7,5: 40% à 90%
   - 6 heures à 6,8 ≤ pH ≤ 7,5: > à 70%

les pourcentages étant donnés en poids de principe actif dissous par rapport au poids total avant dissolution.

Les microgranules utiles dans la forme selon l'invention sont tous avantageusement constitués par un support granulaire neutre enrobé par une couche active constituée par un mélange de principe actif, dérivé de 5-nitro imidazole, et d'un agent liant.

Le support neutre est de manière avantageuse constitué par des particules d'amidon ou d'un mélange d'amidon et de saccharose, d'un diamètre moyen compris entre 400 et 800 microns.

Le dérivé de 5-nitro imidazole est avantageusement choisi parmi le métronidazole, le secnidazole, le tinidazole et leurs mélanges.

L'agent liant est un agent usuel connu pour son utilisation dans la préparation des microganules, avantageusement choisi parmi la polyvinyl-pyrrolidone de différents poids moléculaires (préférentiellement grades K30 et K17 Kollidon (fabriqué par BASF); le grade correspond à la valeur de la constante K, qui est fonction du poids moléculaire et de la viscosité du produit. La constante K est l'objet de spécifications dans les différentes monographies officielles en vigueur, en ce qui concerne la polyvinylpyrrolidone. l'hydroxypropyl-méthylcellulose de différents poids moléculaires (préférentiellement grade 615; les grades HPMC sont fonctions du taux de substitution du produit en groupements Methoxy et Hydroxypropoxy et reflètent la viscosité. Les différents grades sont décrits dans les monographies officielles de ce produit, en particulier à l'USP XXII). l'hydroxy-propyl-cellulose de différents poids moléculaires, les esters poly(méth)acryliques (commercialisés par RÖHM GmbH sous la marque EUDRAGIT®), et leurs mélanges.

Bien entendu, la présente invention s'applique également aux microgranules comprenant un noyau simplement constitué par le principe actif et un agent liant.

Les microgranules gastro-résistants et à libération prolongée se distinguent par leur couche externe d'enrobage de la couche active. Il s'agira d'une part d'une couche externe gastro-résistante pour les microgranules gastro-résistants et d'autre part d'une couche externe de libération prolongée pour les microgranules à libération prolongée.

La couche externe gastro-résistante est constituée par les excipients usuels employés dans la technique pour la préparation de microgranules gastro-résistants. Elle comprend des excipients assurant la résistance de l'enrobage à des pH inférieurs à 5,0, avantageusement choisis parmi les esters poly-(méth)acryliques (commercialisés par RÖHM sous les marques EUDRAGIT® L 100-55, EUDRAGIT® L 100, EUDRAGIT® L 30D-55), l'hydroxypropyl-méthylcellulose phtalate (commercialisé par la société SHIN ETSU Chemical Co., Ltd. sous les marques HP 50® et HP 55®), et leurs mélanges.

La couche externe de libération prolongée comprend pour sa part les excipients usuels employés dans la technique pour la préparation de microgranules à libération prolongée, indépendants du pH du milieu dans lequel ils agissent, avantageusement choisis parmi les esters poly(méth)acryliques (commercialisés par RÖHM sous les marques EUDRAGIT® RS 100, EUDRAGIT® RS 30D), l'éthylcellulose (comercialisée par FMC Corporation, Philadelphie sous la marque AQUACOAT®), et leurs mélanges. Dans ce cas, c'est le temps de transit des microgranules dans les intestins qui déterminera le moment de libération du principe actif.

La couche externe de libération prolongée peut également comprendre un excipient dépendant du pH du milieu dans lequel les microgranules transitent, en particulier les esters poly-(méth)acryliques pH dépendants (commercialisés par RÖHM sous la marque EUDRAGIT® S). Dans ce cas, ce n'est plus le temps de transit des microgranules qui déterminera le moment de libération du principe actif, mais le pH du milieu dans lequel ils transitent. Ainsi, pour assurer la libération du principe actif dans la partie basse des intestins on choisira un excipient soluble à des pH suppérieurs à 7 tels que l'EUDRAGIT® S. Bien entendu, selon l'effet recherché, les microgranules à libération prolongée pourront également consister en un mélange de microgranules à libération dépendante du pH, pour libérer le principe actif à des pH différents, ou encore en un mélange de microgranules dépendant et ne dépendant pas du pH, modulant une libération en fonction du temps de transit et du pH.

La couche externe gastro-résistante et la couche externe de libération prolongée peuvent également comprendre des additifs usuels, tels que du talc qui facilite l'enrobage par ses propriétés lubrifiantes ou des dérivés de la silice ou de l'acide stéarique, et/ou un ou plusieurs plastifiants qui facilitent la bonne formation du film d'enrobage, notamment des esters d'acides (poly)carboxyliques tels que les esters de l'acide citrique (notamment le triéthyl citrate), le dibutyl sébacate et leurs mélanges.

Compte-tenu de l'importance de la taille des microgranules sur leur vitesse de transit dans les intestins, le diamètre moyen des microgranules utiles pour la forme selon l'invention, est avantageusement compris entre 0,4 et 1,5 mm, de préférence compris entre 0,8 et 1,1 mm.

Les microgranules utiles dans la forme selon l'invention sont avantageusement préparées selon le schéma général suivant:
- enrobage du noyau neutre par la couche active,
- enrobage de la couche active soit par une couche gastrorésistante, soit par une couche de libération prolongée selon les microgranules,
- tamisage, et
- séchage.

Les microgranules sont alors mélangés selon les proportions recherchées et conditionnés dans une forme appropriée à son administration, en particulier sous une forme de comprimés, de comprimés à délitement rapide, de gélules ou encore en sachets.

La présente invention concerne donc une composition pharmaceutique comprenant la forme décrite ci-dessus.

Elle concerne également, à titre de produits intermédiaires dans la préparation de la forme galénique selon l'invention, les microgranules gastro-protégés et à libération prolongée décrits plus haut.

Bien entendu, dans la mesure où les microgranules gastrorésistants et à libération prolongée sont préparés et peuvent être conditionnés séparément, la présente invention concerne également un produit de combinaison comprenant des microgranules de dérivés de 5-nitro imidazole gastroprotégés et des microgranules de dérivés de 5-nitro imidazole à libération prolongée tels que décrits ci-dessus pour une utilisation en combinaison simultanée, séparée ou décalée dans le temps pour le traitement des parasitoses et les infections du tractus gastrointestinal, en particulier les amibiases et les infections liées à la présence de *Helicobacter pilori*.

D'autres caractéristiques de la nouvelle forme selon l'invention apparaîtront à la lumière des exemples ci-après.

### Exemple 1: mélange de microgranules de métronidazole 2/8

Le mélange suivant a été préparé:

| | A % | B % |
|---|---|---|
| Métronidazole | 63,8 | 64,5 |
| Noyau neutre | 21,3 | 21,5 |
| PVP K30 | 5,7 | 5,7 |
| HP50 | 9,2 | 4,7 |
| Ethyl cellulose N7 | - | 1,8 |
| Talc | - | 1,8 |
| | 100,0 | 100,0 |

Les pourcentages sont donnés en poids par rapport au poids total des microgranules. Ingrédients:
- Noyau neutre: composé de Saccharose (environ 75%) et d'amidon de maïs (environ 25%)
- PVP : Polyvinylpyrrolidone K 30
- HP 50: Hydroxypropylméthylcellulose Phtalate, dissolution à partir de pH 5,0.
- Ethylcellulose N7: Ethylcellulose dont le grade N7 représente la viscosité du produit.

Les microgranules ont été préparés selon le mode opératoire décrit plus loin.

### Exemple 2: mélange de microgranules de métronidazole 3/7

Le mélange suivant a été préparé selon le même mode opératoire:

| | A % | B % |
|---|---|---|
| Métronidazole | 52,6 | 56,9 |
| Noyau neutre | 19,9 | 21,6 |
| PVP K30 | 2,7 | 2,8 |
| EUDRAGIT® L30D-55 | 11,8 | - |
| Triéthyl citrate | 1,2 | 3,7 |
| Talc | 11,8 | 7,5 |
| EUDRAGIT® S | - | 7,5 |
| | 100,0 | 100,0 |

Les pourcentages sont donnés en poids par rapport au poids total des microgranules.Ingrédients:
- Noyau neutre: composé de Saccharose (environ 75%) et d'amidon de maïs (environ 25%)
- PVP : Polyvinylpyrrolidone K30
- EUDRAGIT® L30D-55: Dispersion aqueuse de copolymères de l'acide Méthacrylique de type C, Dissolution à partir de pH 5,5.
- Talc
- EUDRAGIT® S: copolymère de l'acide Méthacrylique de type B, Dissolution à partir de pH 7,0

### Exemple 3: mélange de microgranules de métronidazole 25/75

Le mélange suivant a été préparé selon le même mode opératoire:

| | A % | B % |
|---|---|---|
| Métronidazole | 51,8 | 59,7 |
| Noyau neutre | 19,6 | 22,6 |
| EUDRAGIT® E 100 | 3,4 | 3,9 |
| EUDRAGIT® L30D-55 | 12,0 | - |
| EUDRAGIT® RS 30D | - | 6,3 |
| Triéthyl citrate | 1,2 | 1,2 |
| Talc | 12,0 | 6,3 |
| | 100,0 | 100,0 |

Les pourcentages sont donnés en poids par rapport au poids total des microgranules.Ingrédients:
- Noyau neutre:composé de Saccharose (environ 75%) et d'amidon de maîs (environ 25%)
- EUDRAGIT® E 100: copolymère de l'acide Méthacrylique utilisé ici en temps que liant.
- EUDRAGIT® L30D-55
- EUDRAGIT® RS 30D: Dispersion aqueuse de copolymères de l'acide Méthacrylique de type B. Dissolution libération prolongée.
- Talc

### Exemple 4: mélange correspondant au lot XM285

Le mélange suivant a été préparé selon le même mode opératoire :

### Composition du lot XM 285

- - Métronidazole :: 65,1%
- - Noyau neutre:: 21,7%
- - PVP K30 :: 4,8%
- - Ethylcellulose N7 :: 1,3%
- - HP 50 :: 5,0%
- - Ethylcellulose N7 :: 1,3%
- - Triéthylcitrate :: 0,5%
- - Talc :: 1,6%
100,0%

### Exemple 5 : Formulation Secnidazole

Le mélange suivant a été préparé selon le même mode opératoire.

| | **A** | **B** |
|---|---|---|
| **Secnidazole** | 47,8% | 45,5% |
| **Noyau neutre** | 33,0% | 31,9% |
| **PVP K17** | 3,4% | 3,2% |
| EUDRAGIT® **L30D-55** | 12,6% | --- |
| EUDRAGIT® **RS30D** | --- | 10,5% |
| **Triéthylcitrate** | 1,3% | 2,1% |
| **Talc** | 1,9% | 6,8% |
| | 100,0% | 100,0% |
| Mélange 25% de A et 75% de B | | |

### Profils de dissolution

Des essais de dissolution des formes des exemples 1 et 4 ont été réalisés à dans une solution de HCl O,1N et à pH 6,8. Les résultats sont reportés sur le tableau ci-dessous.Les pourcentages sont donnés en poids de microgranules dissous par rapport au poids total de microgranules avant dissolution.

| | Exemple 1 | Exemple 4 |
|---|---|---|
| **Essai de gastro-résistance** | | |
| HCl 0,1N pendant 2 heures | 6,7% | 8,5% |

| **Essai de dissolution pH 6,8** | | |
|---|---|---|
| 1 heure | 42,2% | 47,2% |
| 4 heures | 78,2% | 72,3% |
| 6 heures | 86,9 % | 81,5 % |

### Essais cliniques

Une étude clinique de l'efficacité de la forme selon l'invention a été réalisée sur 60 patients des deux sexes, agés de 15 à 75 ans et atteints d'amibiase présentant des formes kystiques dans le colon. L'étude a été réalisée selon une méthodologie rigoureuse en double aveugle, double placébo, "randomisée", en comparant la forme de l'exemple 1 au métronidazole commercialisé sous la marque Flagyl.

Les doses administrées ont été de 1,5 g/jour (3 prises quotidiennes de deux gélules dosées à 250 mg en métronidazole) pendant 10 jours.

Les résultats de cette étude montrent un taux de guérison de 85 % pour la forme selon l'invention contre 14 % pour le Flagyl.

### Protocole de fabrication des microgranules de métronidazole (lot XM 415/2)

### Phase 1 : préparation de la solution liante : solution alcoolique de PVP à 20% dans l'alcool

- la solution est préparée dans un mélangeur inox,
- verser l'alcool éthylique 95% dans le mélangeur,
- mettre en agitation et incorporer par petites quantités le PVP,
- maintenir l'agitation jusqu'à dissolution complète.

### Phase 2 : application

- mettre les grains supports neutres dans la turbine en rotation,
- l'application est réalisée par poudrage du principe actif sur les microgranules neutres, en alternance avec des séquences de pulvérisation de la solution liante,
- tamiser la masse de microgranules, (tamis utilisables : grilles d'ouverture 0,50; 0,71 0,99; 1,12; 1,25 mm),
- sécher les microgranules par insufflation d'air chaud à l'intérieur de la turbine en rotation.

### Phase 3 : séparation de la masse en deux parties

- diviser la masse en deux parties A et B correspondant respectivement à 75% et 25% de la masse de microgranules,
- placer chacune des deux parties dans une turbine différente.

### Phase 4 : préparation de la solution de prémontage : solution alcoolique d'éthylcellulose N7 à 10%

- la solution est préparée dans un mélangeur inox,
- verser l'alcool éthylique 95% dans le mélangeur,
- mettre en agitation et incorporer par petites quantités l'Ethylcellulose
- maintenir l'agitation jusqu'à dissolution complète.

### Phase 5 : prémontage de la partie A

- appliquer par pulvérisation la solution de prémontage sur les microgranules,
- saupoudrer simultanément le talc,
- tamiser la masse de microgranules, (tamis utilisables : grilles d'ouverture 0,50; 0,71 0,99; 1,12; 1,25 mm)
- sécher les microgranules par insufflation d'air chaud à l'intérieur de la turbine en rotation.

### Phase 6 : préparation de la solution d'enrobage : solution acétoalcoolique (20/80) de HP 50 à 7,5%

- la solution est préparée dans un mélangeur inox,
- verser l'alcool éthylique 95% puis l'acétone dans le mélangeur,
- mettre en agitation et incorporer par petites quantités le HP 50,
- maintenir l'agitation jusqu'à dissolution complète.

### Phase 7 : enrobage des parties A et B : l'enrobage est effectué de façon décalée sur les parties A et B

- appliquer par pulvérisation la solution d'enrobage sur les microgranules,
- tamiser la masse de microgranules,
- (tamis utilisables : grilles d'ouverture 0,50 ; 0,71 ; 0,99 ; 1,12 ; 1,25 mm)
- sécher les microgranules par insufflation d'air chaud à l'intérieur de la turbine en rotation

### Phase 8 : lubrification - mélange des deux parties

- introduire les deux parties A et B ainsi que le talc de lubrification dans la turbine en rotation,
- brasser la masse,
- arrêter la turbine.

La nouvelle forme galénique selon l'invention s'avère également efficace dans le traitement préventif des infections à germes anaérobies lors des interventions chirurgicales comportant un haut risque de survenue de ce type d'infections, comme par exemple en chirurgie digestive, lors d'interventions pour des diverticulites, des résections du colon, etc.

## Revendications

1. Forme galénique de dérivés de 5-nitro imidazole caractérisée en ce qu'elle comprend une association de microgranules de dérivés de 5-nitro imidazole, consistant d'une part en des microgranules gastro-résistants et d'autre part en des microgranules à libération prolongée.

2. Forme galénique selon la revendication 1, caractérisée en ce que le rapport pondéral microgranules gastro-résistants/ microgranules à libération prolongée est compris entre 6/4 et 1/9

3. Forme galénique selon la revendication 2, caractérisée en ce que le rapport pondéral microgranules gastro-résistants/microgranules à libération prolongée est compris entre 25/75 et 15/85.

4. Forme galénique selon l'une des revendications 1 à 3, caractérisée en ce que les microgranules associés répondent aux profils de dissolution suivants :
- microgranules gastro-résistants :
2 heures dans HCl 0,1N < à 15%
1 heure à pH 6,0 > à 75 %
- microgranules à libération prolongée :
2 heures dans HCl 0,1 N < à 15%
1 heure à 6,8 ≤ pH ≤ 7,5 < à 50%
4 heures à 6,8 ≤ pH ≤ 7,5 40% à 90%
6 heures à 6,8 ≤ pH ≤ 7,5 > à 70%
les pourcentages étant donnés en poids de microgranules dissous par rapport au poids total avant dissolution.

5. Forme galénique selon l'une des revendications 1 à 4, caractérisée en ce que les microgranules gastro-résistants sont constitués par un support granulaire neutre, enrobé par une couche active constituée par un mélange de dérivé de 5-nitro imidazole et d'un agent liant, et d'une couche externe gastro-résistante.

6. Forme galénique selon la revendication 5, caractérisée en ce que la couche externe gastro-résistante comprend des excipients assurant la résistance de l'enrobage à des pH compris entre 4,5 et 5,5, avantageusement choisis parmi les esters poly-(méth)acryliques, l'hydroxypropyl-méthylcellulose et leurs mélanges, et éventuellement du talc et/ou un ou plusieurs plastifiants.

7. Forme galénique selon l'une des revendications 1 à 4, caractérisée en ce que les microgranules à libération prolongée sont constitués par un support granulaire neutre, enrobé par une couche active constituée par un mélange de dérivé de 5-nitro imidazole et d'un agent liant, et d'une couche externe de libération prolongée.

8. Forme galénique selon la revendication 7, caractérisée en ce que la couche externe de libération prolongée comprend des excipients usuels employés dans la technique pour la préparation de microgranules à libération prolongée, indépendants du pH du milieu dans lequel ils agissent, avantageusement choisis parmi les esters poly-(méth)acryliques, l'éthylcellulose, et leurs mélanges, et éventuellement du talc et/ou un ou plusieurs plastifiants.

9. Forme galénique selon la revendication 8, caractérisée en ce que la couche externe gastro-résistante comprend au moins un excipient dépendant du pH du milieu dans lequel les microgranules transitent, en particulier les esters poly-(méth)acryliques pH dépendants, et éventuellement du talc et/ou un ou plusieurs plastifiants.

10. Forme galénique selon l'une des revendications 5 à 9, caractérisée en ce que le support neutre est constitué par des particules d'amidon ou d'un mélange d'amidon et de Saccharose, d'un diamètre moyen compris entre 400 et 800 microns.

11. Forme galénique selon l'une des revendications 5 à 10, caractérisée en ce que l'agent liant est un agent usuel connu pour son utilisation dans la préparation des microgranules, avantageusement choisi parmi le polyvinyl-pyrrolidone de différents poids moléculaires, l'hydroxypropyl-méthylcellulosede différents poids moléculaires, l'hydroxypropylcellulose de différents poids moléculaires, les esters poly-(méth)acryliques et leurs mélanges.

12. Forme galénique selon l'une des revendications 1 à 11, caractérisée en ce que les microgranules gastro-résistants et à libération prolongée ont un diamètre moyen compris entre 0,4 et 1,5 mm, de préférence compris entre 0,8 et 1,1 mm.

13. Forme galénique selon l'une des revendications 1 à 12, caractérisé en ce que le dérivé de 5-nitro imidazole est choisi parmi le métronidazole, le secnidazole, le tinidazole et leurs mélanges.

14. Compositions pharmaceutiques caractérisée en ce qu'elle comprend la forme galénique selon l'une des revendications 1 à 13, en particulier sous la forme de comprimés, de comprimés à délitement rapide ou de gélules.

15. Microgranules gastro-protégés de dérivés de 5-nitro imidazole, utiles notamment pour la préparation de la forme galénique selon l'une des revendications 1 à 13.

16. Microgranules à libération prolongée de dérivés de 5-nitro imidazole, utiles notamment pour la préparation de la forme galénique selon l'une des revendications 1 à 13.

17. Produit de combinaison comprenant des microgranules de dérivés de 5-nitro imidazole gastro-protégé et des microgranules de dérivés de 5-nitro imidazole à libération prolongée, pour leur utilisation en combinaison simultanée, séparée ou décalée dans le temps pour le traitement des parasitoses et/ou des infecions du tractus gastro-intestinal, en particulier les amibiases et les infections liées à la présence de *Hélicobacter pilori*.

## Claims

1. Galenic form of 5-nitroimidazole derivatives characterized in that it comprises a combination of microgranules of 5-nitroimidazole derivatives consisting, on the one hand, of gastroresistant microgranules and, on the other hand, of prolonged-release microgranules.

2. Galenic form according to Claim 1, characterized in that the gastroresistant microgranule/prolonged-release microgranule weight ratio is between 6/4 and 1/9.

3. Galenic form according to Claim 2, characterized in that the gastroresistant microgranule/prolonged-release microgranule weight ratio is between 25/75 and 15/85.

4. Galenic form according to one of Claims 1 to 3, characterized in that the combined microgranules should correspond to the following dissolution patterns:
- gastroresistant microgranules:
2 hours in 0.1 N HCl < 15%
1 hour at pH 6.0 > 75%
- prolonged-release microgranules:
2 hours in 0.1 N HCl < 15%
1 hour at 6.8 ≤ pH ≤ 7.5 < 50%
4 hours at 6.8 ≤ pH ≤ 7.5 40% to 90%
6 hours at 6.8 ≤ pH ≤ 7.5 >70%
the percentages being given by weight of microgranules dissolved relative to the total weight before dissolution.

5. Galenic form according to one of Claims 1 to 4, characterized in that the gastroresistant microgranules consist of a neutral granular carrier coated with an active layer consisting of a mixture of 5-nitroimidazole derivative and a binding agent, and of a gastroresistant external layer.

6. Galenic form according to Claim 5, characterized in that the gastroresistant external layer comprises excipients ensuring the resistance of the coating to pH values of between 4.5 and 5.5, advantageously chosen from poly(meth)acrylic esters, hydroxypropyl methylcellulose and mixtures thereof, and optionally talc and/or one or more plasticizers.

7. Galenic form according to one of Claims 1 to 4, characterized in that the prolonged-release microgranules consist of a neutral granular carrier coated with an active layer consisting of a mixture of 5-nitroimidazole derivative and a binding agent, and of a prolonged-release external layer.

8. Galenic form according to Claim 7, characterized in that the prolonged-release external layer comprises customary excipients used in the technique for the preparation of prolonged-release microgranules, independent of the pH of the medium in which they act, advantageously chosen from poly(meth)acrylic esters, ethyl cellulose and mixtures thereof, and optionally talc and/or one or more plasticizers.

9. Galenic form according to Claim 8, characterized in that the gastroresistant external layer comprises at least one excipient which is dependent on the pH of the medium in which the microgranules transit, in particular pH-dependent poly(meth)acrylic esters, and optionally talc and/or one or more plasticizers.

10. Galenic form according to one of Claims 5 to 9, characterized in that the neutral carrier consists of particles of starch or of a mixture of starch and sucrose which have an average diameter of between 400 and 800 microns.

11. Galenic form according to one of Claims 5 to 10, characterized in that the binding agent is a customary agent known for its use in the preparation of microgranules, advantageously chosen from polyvinylpyrrolidone of various molecular weights, hydroxypropyl methylcellulose of various molecular weights, hydroxypropyl cellulose of various molecular weights, poly(meth)acrylic esters and mixtures thereof.

12. Galenic form according to one of Claims 1 to 11, characterized in that the gastroresistant and prolonged-release microgranules have an average diameter of between 0.4 and 1.5 mm, preferably of between 0.8 and 1.1 mm.

13. Galenic form according to one of Claims 1 to 12, characterized in that the 5-nitroimidazole derivative is chosen from metronidazole, secnidazole, tinidazole and mixtures thereof.

14. Pharmaceutical composition characterized in that it comprises the galenic form according to one of Claims 1 to 13, in particular in the form of tablets, of rapidly-disintegrating tablets or gelatin capsules.

15. Gastro-protected microgranules of 5-nitroimidazole derivatives which are useful especially for the preparation of the galenic form according to one of Claims 1 to 13.

16. Prolonged-release microgranules of 5-nitroimidazole derivatives which are useful especially for the preparation of the galenic form according to one of Claims 1 to 13.

17. Combination product comprising microgranules of gastro-protected 5-nitroimidazole derivatives and microgranules of prolonged-release 5-nitroimidazole derivatives for use as a combination simultaneously, separately or spaced out over time for the treatment of parasitoses and infections of the gastrointestinal tract, in particular amoebiases and infections linked to the presence of *Helicobacter pilori*.

## Patentansprüche

1. Galenische Form von 5-Nitro-imidazol-Derivaten, dadurch charakterisiert, daß sie eine Kombination von Mikrogranulaten von 5-Nitro-imidazol-Derivaten umfaßt, bestehend einerseits aus magensaftresistenten Mikogranulaten und andererseits aus Mikrogranulaten mit verzögerter Freisetzung.

2. Galenische Form nach Anspruch 1, dadurch charakterisiert, daß das Gewichtsverhältnis magensaftresistente Mikrogranulate/Mikrogranulate mit verzögerter Freisetzung zwischen 6/4 und 1/9 liegt.

3. Galenische Form nach Anspruch 2, dadurch charakterisiert, daß das Gewichtsverhältnis magensaftresistente Mikrogranulate/Mikrogranulate mit verzögerter Freisetzung zwischen 25/75 und 15/85 liegt.

4. Galenische Form nach einem der Ansprüche 1 bis 3, dadurch charakterisiert, daß die kombinierten Mikrogranulate den folgenden Lösungsprofilen entsprechen:
- magensaftresistente Mikrogranulate:
2 Stunden in HCl 0,1 N < 15%
1 Stunde bei pH 6,0 > 75 %
- Mikrogranulate mit verzögerter Freisetzung:
2 Stunden in HCl 0,1 N < 15 %
1 Stunde bei 6,8 ≤ pH ≤ 75 < 50 %
4 Stunden bei 6,8 ≤ pH ≤ 7,5 40 % bis 90 %
6 Stunden bei 6,8 ≤ pH ≤ 75 >70 %
wobei die Prozentsätze angegeben sind als Gewicht von gelösten Mikrogranulaten, bezogen auf das Gesamtgewicht vor dein Lösen.

5. Galenische Form nach einem der Ansprüche 1 bis 4, dadurch charakterisiert, daß die magensaftresistenten Mikrogranulate aufgebaut sind aus einem körnigen neutralen Träger, der beschichtet ist mit einer aktiven Schicht, die aus einer Mischung von 5-Nitro-imidazol-Derivat und einem Bindemittel aufgebaut ist, sowie mit einer äußeren magensaftresistenten Schicht.

6. Galenische Form nach Anspruch 5, dadurch charakterisiert, daß die äußere magensaftresistente Schicht Exzipienten umfaßt, die die Beständigkeit der Beschichtung bei pH-Werten zwischen 4,5 und 5,5 gewährleistet, vorteilhafterweise ausgewählt aus Poly(meth)acrylsäureestern, Hydroxypropylmethylcellulose und ihren Mischungen, und gegebenenfalls Talkum und/oder einen oder mehrere Weichmacher.

7. Galenische Form nach einem der Ansprüche 1 bis 4, dadurch charakterisiert, daß die Mikrogranulate mit verzögerter Freisetzung aufgebaut sind aus einem neutralen körnigen Träger, der beschichtet ist mit einer aktiven Schicht, die aus einer Mischung von 5-Nitro-imidazol-Derivat und einem Bindemittel aufgebaut ist, sowie einer äußeren Schicht mit verzögerter Freisetzung.

8. Galenische Form nach Anspruch 7, dadurch charakterisiert, daß die äußere Schicht mit verzögerter Freisetzung übliche in der Technik für die Herstellung von Mikrogranulaten mit verzögerter Freisetzung verwendete Exzipienten umfaßt, die unabhängig sind vom pH des Mediums, in dem sie sich befinden, vorteilhafterweise ausgewählt aus Poly(meth)acrylsäureestern, Ethylcellulose und ihren Mischungen, und gegebenenfalls Talkum und/oder einen oder mehrere Weichmacher.

9. Galenische Form nach Anspruch 8, dadurch charakterisiert, daß die äußere magensaftresistente Schicht mindestens einen Exzipienten umfaßt, der abhängig vom pH des Mediums ist, in das Mikrogranulate übergehen, insbesondere pH-abhängige Poly(meth)acrylsäureester, und gegebenenfalls Talkum und/oder einen oder mehrere Weichmacher.

10. Galenische Form nach einem der Ansprüche 5 bis 9, dadurch charakterisiert, daß der neutrale Träger gebildet wird aus Partikeln aus Stärke oder einer Mischung von Stärke und Saccharose mit einem mittleren Durchmesser zwischen 400 und 800 µm.

11. Galenische Form nach einem der Ansprüche 5 bis 10, dadurch charakterisiert, daß das Bindemittel ein übliches Mittel ist, das für seine Verwendung bei der Herstellung von Mikrogranulaten bekannt ist, vorteilhafterweise ausgewählt aus Polyvinylpyrrolidon mit verschiedenen Molekulargewichten, Hydroxypropylmethylcellulose mit verschiedenen Molekulargewichten, Hydroxypropylcellulose mit verschiedenen Molekulargewichten, Poly(meth)acrylsäureestern und ihren Mischungen.

12. Galenische Form nach einem der Ansprüche 1 bis 11, dadurch charakterisiert, daß die magensaftresistenten Mikrogranulate und die Mikrogranulate mit verzögerter Freisetzung einen mittleren Durchmesser zwischen 0,4 und 1,5 mm aufweisen, bevorzugt zwischen 0,8 und 1,1 mm.

13. Galenische Form nach einem der Ansprüche 1 bis 12, dadurch charakterisiert, daß das 5-Nitro-imidazol-Derivat ausgewählt wird aus Metronidazol, Secnidazol und ihren Gemischen.

14. Pharmazeutische Zusammensetzungen, dadurch charakterisiert, daß sie die galenische Form nach einem der Ansprüche 1 bis 13 umfassen, insbesondere in Form von Tabletten, schnell zerfallenden Tabletten oder Gelkapseln.

15. Magensaftresistente Mikrogranulate von 5-Nitro-imidazol-Derivaten, die insbesondere nützlich sind für die Herstellung der galenischen Form nach einem der Ansprüche 1 bis 13.

16. Mikrogranulate mit verzögerter Freisetzung von 5-Nitro-imidazol-Derivaten, die insbesondere nützlich sind für die Herstellung der galenischen Form nach einem der Ansprüche 1 bis 13.

17. Kombinationsprodukt, umfassend magensaftresistente Mikrogranulate von 5-Nitroimidazol-Derivaten und Mikrogranulate mit verzögerter Freisetzung von 5-Nitroimidazol-Derivaten zur Verwendung in gleichzeitiger, getrennter oder aufeinanderfolgender Kombination für die Behandlung von Parasitosen und/oder Infektionen des Gastrointestinal-Trakts, insbesondere Amöbiasen und mit dem Vorhandensein von Helicobacter pylori zusammenhängenden Infektionen.
